# EUROPEAN PATENT APPLICATION

(11) **EP 3 381 511 A1**
(43) Date of publication of application: **03.10.2018**
(21) Application number: 17305367.9
(22) Date of filing: 30.03.2017
(51) Int. Cl.: A61N 5/06

(54) **DIRECTLY MODULATED LASERS FOR OPTOGENETIC THERAPY**

(71) Applicant: Nokia Solutions and Networks Oy, 02610 Espoo (FI)
(72) Inventor: GARREAU, Alexandre, 91767 Palaiseau (FR); BRENOT, Romain, 91767 Palaiseau (FR); ACHOUCHE, Mohand, 91767 Palaiseau (FR)
(74) Representative: Novagraaf Technologies

(57) **Abstract**

The present invention relates to apparatuses for stimulating *in vivo* light-sensitive cells of an area comprising at least a light source having a wavelength adapted to stimulate the light-sensitive cells.

They can be used on muscles or organs requiring an excitation and inhibition like heart suffering cardiomyopathy.

Such apparatuses provide a homogeneous stimulation of the light-sensitive cells of an area that can be larger than the area covered by a traditional electrode.

## Description

### SPECIFICATION

### Field of the Invention

The present invention relates to a device for stimulating *in vivo* light-sensitive cells or proteins especially for optogenetic therapy in cardiomyopathy disease or on muscles or organs requiring excitation and inhibition.

### Background

The heart exhibits remarkable adaptive responses to a wide array of genetic and extrinsic factors to maintain contractile function. When compensatory responses are not sustainable, cardiac dysfunction occurs, leading to cardiomyopathy. A wide range of inherited and acquired cardiomyopathies has been described by the World Health Organization: hypertrophic cardiomyopathy, dilated cardiomyopathy, restrictive cardiomyopathy, and arrhythmogenic right ventricular cardiomyopathy as well as a large group of unclassified cardiomyopathies. Acquired cardiomyopathies induced by stress, diabetes, some chemotherapeutic agents, pregnancy, and alcohol intake, for example, represent a diverse class of cardiac disease.

Since 1958, the artificial pacemaker is a medical device which uses electrical impulses, delivered by electrodes contracting the heart muscles, to regulate the beating of the heart. The primary purpose of a pacemaker is to maintain an adequate heart rate, either because the heart's natural pacemaker is not fast enough, or because there is a block in the heart's electrical conduction system. Modern pacemakers are externally programmable and allow a cardiologist to select the optimum pacing modes for individual patients. Some combine a pacemaker and defibrillator in a single implantable device. Others have multiple electrodes stimulating differing positions within the heart to improve synchronisation of the lower chambers (ventricles) of the heart.

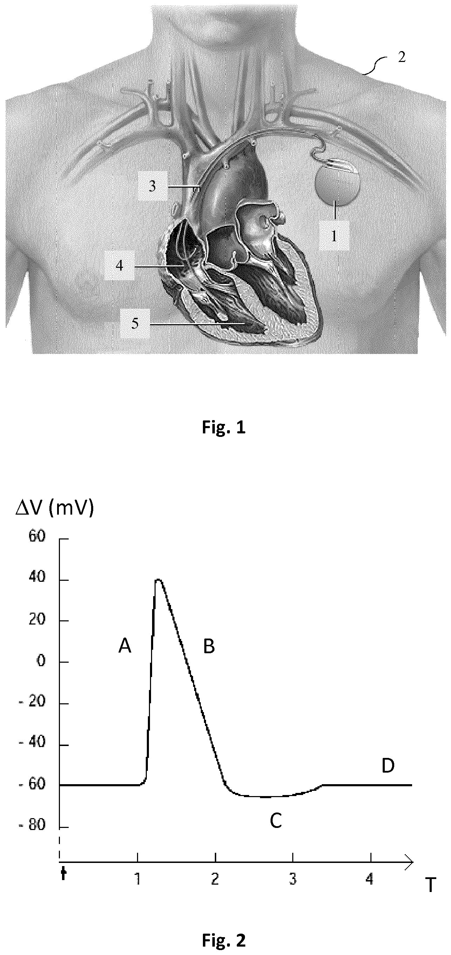

**Figure 1** illustrates an artificial pacemaker 1 implanted in a body 2. The artificial pacemaker 1 comprises two electrode leads 3, one going into the right atrium 4 and the other into the right ventricle 5. The artificial pacemaker 1 controls the pacing heart rate and the output voltage of the electrode leads that stimulate the heart muscles and so the heart beatings.

However, such devices, the electrical stimulations are located around the output of the electrode leads so the stimulation is not homogeneous which can cause local inflammation.

Another solution is thus currently studied by the inventors based on optogenetic therapy researches.

### Summary

The present invention proposes apparatuses for stimulating *in vivo* light-sensitive cells or proteins to solve the prior art drawbacks.

In a first and general apparatus embodiment, the apparatus comprises two devices, each devices comprising a light source, the wavelength of the light source been adapted to stimulate a type of light-sensitive cells or proteins, the first device being adapted to stimulate the first type of light-sensitive cells with a first wavelength λ₁ and the second device being adapted to stimulate the second type of light-sensitive cells with a second wavelength λ₂. Such apparatus makes it possible to stimulate two different type of cells like for cardiomyopathies treatment based on optogenenic technique.

Advantageously, the devices used are diodes formed on the same semiconductor and electrically separated so as to have a simple and compact structure.

To guarantee the biocompatibility of the apparatus, the semiconductor may have active layers encapsulated in a biocompatible material.

Advantageously, the active layers formed a periodic structure of multi quantum wells or layers composed of quantum dots or quantum dashes generating alternatively the light of the first diode and of the second diode, the structure being adapted to optimize the homogeneity of the power density of the light emitted by the first and second diodes.

To prevent electrical interactions / interferences between different quantum wells or quantum dots and quantum dashes, they may be electrically separated.

Advantageously, the semiconductor has a GaN substrate and top layer forming a p-n junction, the substrate and the top layer having opposite doping, in order to supply the optical amplifiers with voltage or current and being able to polarize the device.

Advantageously, the diodes are electrically polarized oppositely so that one signal is adapted to pilot them alternatively.

To regulate the stimulation of the light sensitive cells, particularly with the ones used in cardiomyopathy treatment on person having optogenic therapy, the diodes may generate alternatively periodical pulses.

To be able to modify the parameter of the light source emission, the apparatus may further comprise a control unit adapted to modify the parameter of the signal which pilots the diodes.

Advantageously, the control unit includes an input adapted to receive information coming from a probe and wherein the signal controlling the diodes is modified according to the information received, facilitating the access to the parameters and therefore their modification.

In order to protect the apparatus from the environment, the semiconductor can be insulated by an insulating layer.

The present invention further relates to a muscle stimulator for exciting and inhibiting a muscle having received an optogenetic treatment with two types of light sensitive cells or proteins, comprising an apparatus as previously described, the apparatus being adapted to stimulate the two types of light sensitive cells or proteins so as to excite or inhibit the muscle when one of the types of light sensitive cells or proteins is adapted to stimulate the muscle contraction and the other is adapted to stimulate the muscle relaxation.

The present invention further relates to an artificial pacemaker for treating cardiomyopathy on a person having received an optogenetic treatment with two types of light sensitive cells, the artificial pacemaker comprising an apparatus as previously described, the apparatus being adapted to stimulate heart muscle contraction and others adapted to stimulate the two types of light sensitive cells or proteins so as to reproduce adapted heartbeats when one of the types of light sensitive cells or proteins is adapted to stimulate the heart muscle contraction and the other is adapted to stimulate the heart muscle relaxation.

### Brief Description of the Drawings

Some embodiments of apparatus in accordance with embodiments of the present invention are now described, by way of example only, and with reference to the accompanying drawings, in which :
- The Figure 1 schematically illustrates a current artificial pacemaker.
- The Figure 2 schematically illustrates the different stages of a cell polarization;
- The Figure 3 schematically illustrates the activation of channelrhodopsin-2 (ChR2) and halorhodopsin (NpHR) by blue and yellow light and its effects on human induced pluripotent stem cell-derived cardiomyocytes (hiPSC-CM);
- The Figure 4 schematically illustrates the synchronization of two hiPSC-CM colonies with blue light;
- The Figure 5 schematically illustrates synchronization of two hiPSC-CM colonies with yellow light;
- The Figure 6 schematically illustrates a side view of a vertical design according to a device embodiment;
- The Figures 7-8 schematically illustrate side views of an horizontal design according to a device embodiment;
- The Figures 9-10 schematically illustrate respectively the side and top views of a vertical apparatus embodiment;
- The Figures 11-12 schematically illustrate respectively the side and top views of an horizontal apparatus embodiment;
- The Figure 13 illustrates an electrical assembly of an apparatus embodiment; and
- The Figure 14 illustrates a type of power signal applicable to the electrical assembly.

### Description of Embodiments

Optogenetics is a biological technique which involves the use of light to control cells in living tissue, particularly in neurons. In the case of the heart, in vitro studies have recently been conducted focusing on specific types of cells: cardiac muscle cells or cardiomyocytes.

Human pluripotent stem cells (hPSC), including human embryonic stem cells (hESC) and human induced pluripotent stem cells (hiPSC) can give rise to every cell type in the body and have been differentiated into various electrically active cell types, including cardiomyocytes (hESC-CM) (hiPSC-CM).

In order to understand the functioning of optogenetics, it is important to remember how a cell works and more specifically what depolarization is.

Depolarization is essential to the function of many cells, communication between cells, and the overall physiology of an organism.

The process of depolarization is entirely dependent upon the intrinsic electrical nature of most cells. When a cell is at rest, the cell maintains what is known as a resting potential. The resting potential generated by nearly all cells results in the interior of the cell having a negative charge compared to the exterior of the cell. To maintain this electrical imbalance, microscopic positively and negatively charged particles called ions are transported across the cell membrane. The transport of the ions across the cell membrane is accomplished through several different types of transmembrane proteins embedded in the cell membrane that function as pathways for ions both into and out of the cell, such as ion channels, sodium potassium pumps, and voltage gated ion channels.

The cell membrane, which is the protective envelop of the cell, is the borderline between the intracellular and the extracellular medium that are both electrically charged.

The intracellular medium, the cytoplasm, is negatively charged and presents a majority of potassium ions K+, and the extracellular medium, the interstitial sector (which is used as a sort of "framework" between the cells) is positively charged and presents a majority of chloride ions CI- and sodium ions Na+.

Each cells are constantly polarized because the function of each cells is activated by the polarization which can be divided into four stages: depolarization, repolarization, hyperpolarization and the resting potential.

**Figure 2** illustrates the different stages of a cell polarization.

At to, the cell is at its resting potential then a stimulation is applied to the cell at t₁ (generally an electrical stimulation) which causes a depolarization A of the cell. Sodium ions Na+ enter into the cell through "Na+ channels" which modifies the electrical charge of the cell. The cell is depolarized and becomes positively charges due to a majority presence of sodium ions Na+ than potassium ions K+ within the cell.

Then the cell repolarizes, stage B, to return to its previous equilibrium. Potassium ions K+ come out of the cell through "K+ channels".

The process of repolarization causes an overshoot in the potential of the cell. Potassium ions K+ continue to move out of the cell so much so that the resting potential is exceeded and the new cell potential becomes more negative than the resting potential. This stage is called hyperpolarization C.

The resting potential D is ultimately re-established by the closing of all ion channels.

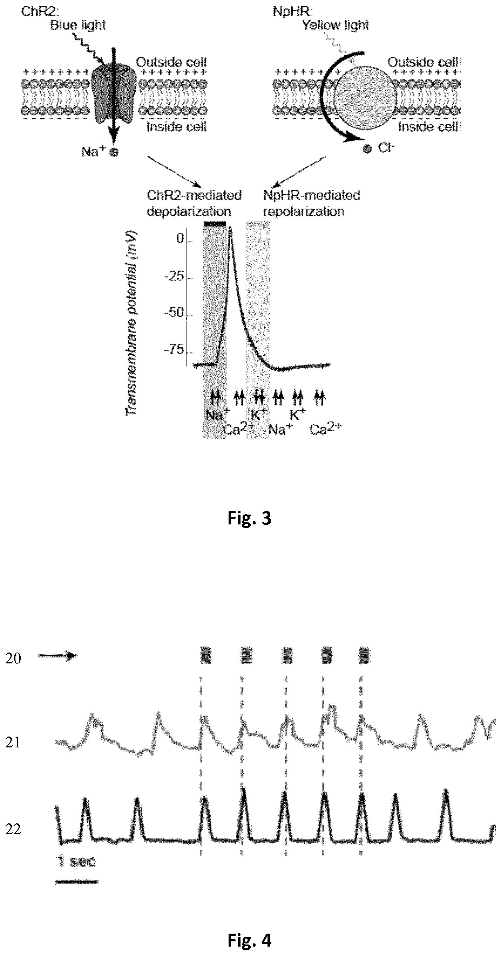

So, optogenetics is a technology based on the targeted genetic introduction of light-sensitive channels (or proteins), such as channelrhodopsin-2 (ChR2), and pumps, such as halorhodopsin (NpHR), into cells. Optogenetic stimulations of hESC-CM by ChR2 and both stimulation and inhibition of hiPSC-CM by ChR2 and NpHR1.0, a first generation halorhodopsin inhibitor, have been demonstrated *in vitro* (see **Figure 3**). **Figure 3** shows that activation of channelrhodopsin-2 (ChR2) and halorhodopsin (NpHR) allows depolarization and repolarization of an action potential in human induced pluripotent stem cell-derived cardiomyocytes (hiPSC-CM).

By stimulation with blue light at a wavelength of 480 nm, hiPSCChR2/NpHR-CM can be activated at frequencies ranging from 0.5 to 1.5 Hz. **Figure 4** shows the synchronization of two hiPSC-CM colonies 21 and 22 with blue light 20 delivered at 1 Hz. The activation of the cells causes a contraction of the cells. It should be noted that the colonies are asynchronous before and after the application of the light pulses.

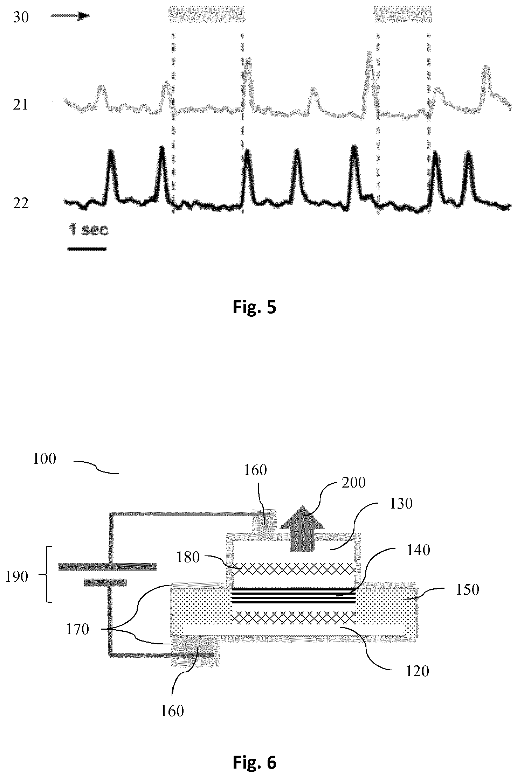

hiPSCChR2/NpHR-CM can also be inhibited with yellow light at a wavelength of 580nm. **Figure 5** shows the synchronization of the two colonies 21 and 22 with yellow light 30. The inhibition of the cells causes the cells to relax.

So the association of periodic activation and inhibition of hiPSCChR2/NpHR-CM cells could, if introduced into the cells of a human heart, regulate the heart beats of someone suffering cardiomyopathy.

According to this principle, the present invention provides optical devices and apparatuses for stimulating optogenetically treated muscles which will overcome the limitation of low spatial control of traditional electrode stimulation, due in part by electrode size and spacing.

In a first and general device embodiment, the invention provides a device for stimulating *in vivo* light-sensitive cells or proteins of an area, the device comprises a light source, and the wavelength of the light source is adapted to stimulate the light-sensitive cells or proteins.

The area treated corresponds to the surface lighted by the device. With divergent light sources, the surface of area treated will depend on the divergence of the light source and the distance between the light source and the area. With a directive light source, like that of a laser diode, the surface of the area treated will depend on the emission surface of the light source, the larger the emission is, the larger the surface treated will be.

So advantageously, the light source is a semiconductor diode which is generally composed of active layers.

In order to be used *in vivo,* the active layers of the semiconductor diode are preferably encapsulated in a biocompatible material like carbon like diamond, Titanium dioxide, common dielectric materials (silica, silicon nitride...) or Gallium nitride (GaN).

In fact, GaN semiconductors present the interest to have a good chemical stability and to be biocompatible. It also has the characteristics to be transparent in the visible range.

Usually, GaN devices are used in ultraviolet range: bulk GaN nanowire photodetectors working in photovoltaic or photoconductive operation modes. Indium Gallium Nitride (InGaN) / GaN multi quantum wells are also well known for blue laser. Moreover, nitride-based photodiodes at 510 nm wavelength have been realized recently.

So, advantageously, the semiconductors include active layers comprising several epitaxies of InGaN and Aluminum Gallium Nitride (AlGaN) or InGaN and Gallium Arsenic Nitride (GaAsN).

Advantageously, the active layers form a multi quantum wells structure or layers composed of quantum dots or quantum dashes adapted to optimize the homogeneity of the power density of the light emitted by the diode.

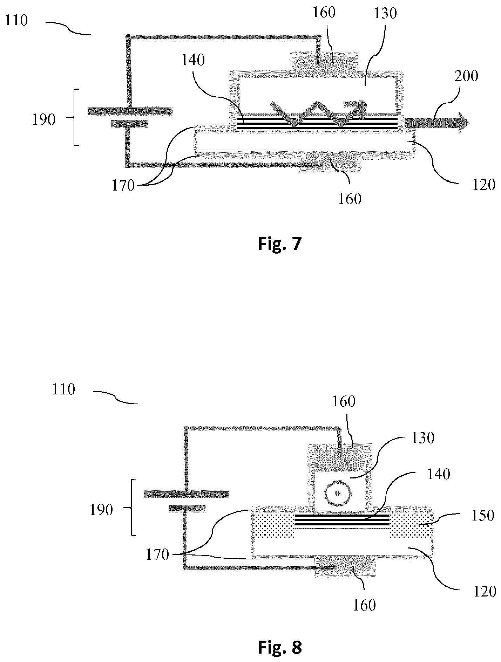

**Figures 6-8** illustrate two different designs of the device: a vertical design 100 or Vertical Cavity Surface Emitting Laser (VCSEL), in **Figure 6** and a horizontal design 110 or Ridge Laser Diode, in **Figures 7-8****.**

The global design is common for both optoelectronic devices 100 and 110. It is based on one or several epitaxies of InGaN/GaNAs or InGaN/AlGaN multi-quantum wells 140 as intrinsic active layer on a GaN Substrate 120. It is possible to define several multi quantum wells structures 140, by using Selective Area Growth technology (SAG) or Butt-joint technology, in order to obtain the stimulating wavelength which depends on the light-sensitive cells or proteins used (ex: blue, green, yellow, etc.).

On the top of the structure, a GaN layer 130 is grown to finish the epitaxy. If the GaN substrate 120 is doped p, the top epitaxied layers 130 are doped n. Reciprocally, if the GaN substrate 120 is doped n, the top epitaxied layers 130 are doped p, so that the substrate and the top layer form a p-n junction. In order to power the semiconductor diode, the two p and n dopings must be implemented and connected to electrical contacts 160 linked to a power source 190.

The different quantum wells on the same array are electrically separated to prevent electrical interactions or interferences. This isolation can be realized by an insulation layer 150, or by implantation of hydrogen ions H+ or by etching to reach the insulated substrate or layer.

The device can also be isolated to protect it from the environment, by an insulating layer 170 that must be a dielectric material or a semi-insulating semiconductor. As specified above, it must have a good biocompatibility like carbon, diamond, Titanium dioxide, common dielectric materials (silica, silicon nitride...) or semi-insulating GaN.

The encapsulation of the active layers by such a biocompatible material enables to use hazardous materials to compose the active layers, while ensuring that the device will still be well received by the organism.

To increase the power of the light emitted, the quantum well generating region 140 can be put between reflecting mirrors 180 forming a laser cavity like two Distributed Bragg Reflectors.

In the vertical design 100 illustrated in **Figure 6****,** the light is generated and then amplified by the quantum well generating region 140 after one or more round-trip in the laser cavity formed by the Distributed Bragg Reflectors 180 before exiting through the top layer 130.

The dimension of the elementary emitter can variate from 1µm to 100µm. This elementary device is the repeated element of a matrix covering the muscle area to be stimulated and/or inhibited. By this way a large area can be treated. Because, in this example, the emitted light 200 goes out of the device through the top layer 130, the metal contact 160 must not cover the entire top layer 130 surface.

In the horizontal design 110 illustrated in **Figures 7-8****,** the light is generated by the quantum well generating region or active layers 140 and then it propagates in the plan of the **Figure 7** and can be amplified by means of a laser cavity before going out of the device.

To regulate and control the emission of the light source so that it generates periodical pulses, for example, the power source can vary in shape, like a square wave signal, frequency and intensity. The periodic shape of the injected electric current by the power source defines the rhythm of the stimulation of the light-sensitive cells or proteins.

In order to cover a larger area and stimulate more light-sensitive cells or proteins, the device can further comprise a lens adapted to widen the beam. Advantageously, the lens is a microlens array where each microlens widen the light beam emitted by a quantum well.

The present invention also relates to an apparatus for stimulating *in vivo* two different type of light-sensitive cells or proteins of an area comprising two devices as one previously described. So, each device comprises at least a light source, the wavelength of the light source being adapted to stimulate a type of light-sensitive cells or proteins. In particular, the first device is adapted to stimulate the first type of light-sensitive cells or proteins with a first wavelength λ₁ and the second device is adapted to stimulate the second type of light-sensitive cells or proteins with a second wavelength λ₂.

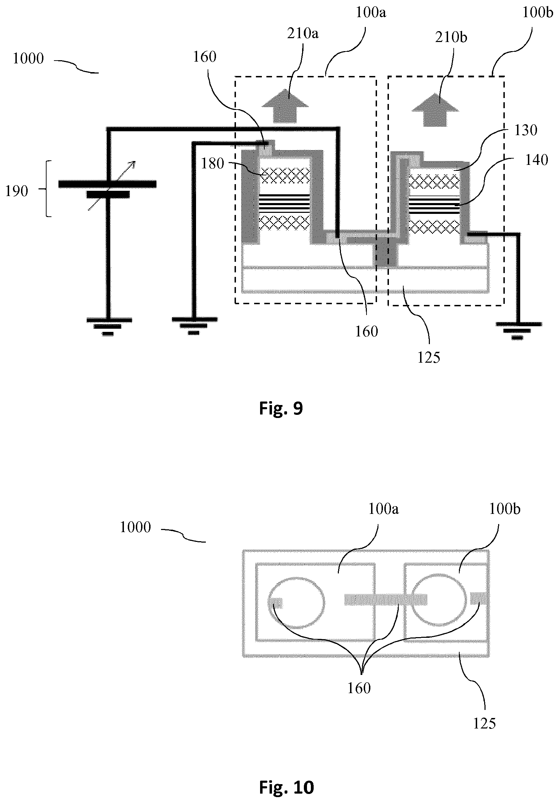

Advantageously, the devices are diodes formed on the same semiconductor and electrically separated as illustrated in **figures 9-12** and the semiconductor can be isolated by an insulating layer to protect it from the environment.

In order to be used in vivo, the semiconductor has active layers encapsulated in a biocompatible material like carbon like diamond, Titanium dioxide, common dielectric materials (silica, silicon nitride...) or Gallium nitride (GaN).

This semiconductor can be made by epitaxy, on a single substrate 125, of active layers 140 forming a periodic structure of multi quantum wells or layers composed of quantum dots or quantum dashes generating alternatively the light of the first diode, λ₁ and of the second diode, λ₂. The structure is adapted to optimize the homogeneity of the power density of the light emitted by the first and second diodes 210a and 210b.

As previously described, the different quantum wells or quantum dots or quantum dashes on the same array can be electrically separated to prevent electrical interactions or interferences. This isolation can be realized by an insulation layer, or by implantation of hydrogen ions H+ or by etching to reach the insulated substrate or layer.

The power density of the light emitted by each diode is controlled by the size of the devices 100a-b and 110a-b, the thickness of the active layers 140, the reflectivity of the mirrors 180 used as laser cavity and the composition of the active layers 140.

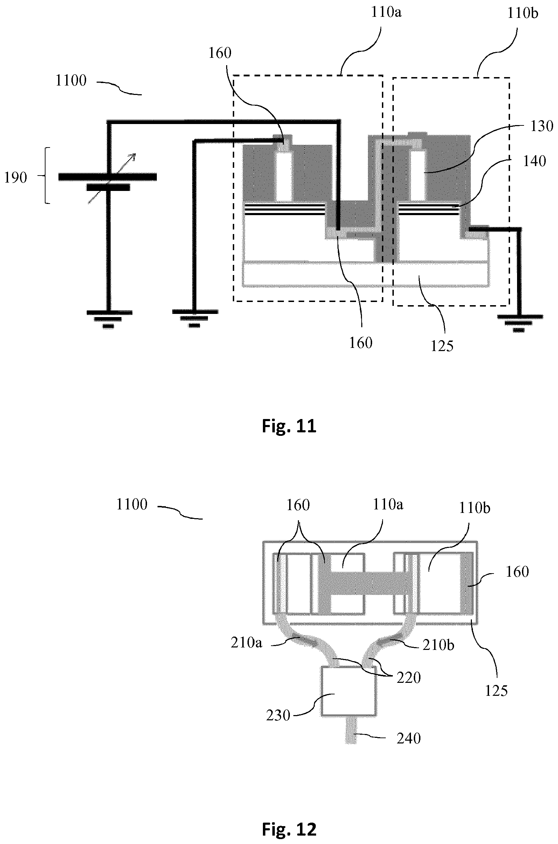

**Figures 9-10** illustrate a vertical embodiment of the apparatus 1000 comprising on the same insulated substrate 125, two devices 100a and 100b in a vertical configuration. **Figure 11-12** illustrate a horizontal embodiment of the apparatus 1100 comprising on the same insulated substrate 125, two devices 110a and 110b in a horizontal configuration.

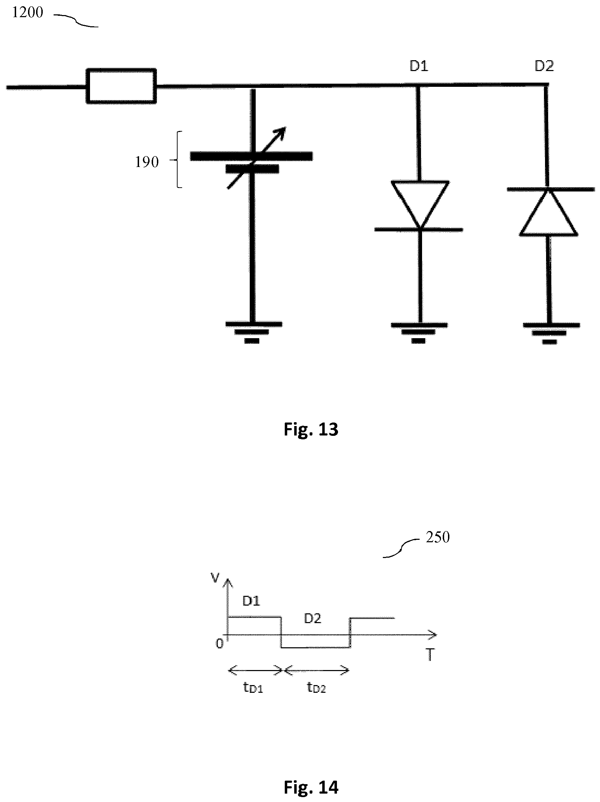

In order to control alternatively both diodes with only one signal, the diodes can be electrically polarized oppositely as illustrated in **figures 9-12****.** In these figures, each devices 100a-b or 110a-b have a common p (or n) substrate and a common n (or p) top layer forming a p-n junction. So to polarized each device oppositely, the negative electrical contacts 160 of the first device 100a or 110a is connected to the positive electrical contact 160 of the second device 100b or 110b (or reciprocally). An example of such an electrical assembly 1200 is illustrated in **figure 13** where D1 represents the first diode and D2 represents the second diode.

**Figure 14** illustrates a type of power signal 250, here a square wave, which can be applied to the electrical assembly 1200. During t_{D1}, a positive voltage is applied to the electrical assembly 1200 so that the first diode D1 emits a light pulse at λ₁ during t_{D1} while the second diode D2 is inhibited. Then a negative voltage is applied so that the second diode D2 emits a light pulse at λ₂ during t_{D2} while the first diode D1 is inhibited, etc. so that the diodes generate alternatively periodical pulses.

Advantageously, the apparatus further comprises a control unit adapted to modify parameters of the signal which pilots the diodes, such as intensity, frequency, form.

Advantageously, the control unit includes an input adapted to receive information coming from a probe and wherein the signal controlling the diodes is modified according to the information received.

The present invention further relates to a muscle stimulator for exciting and inhibiting a muscle having received an optogenetic treatment with two types of light sensitive cells or proteins, comprising an apparatus as previously described, the apparatus being adapted to stimulate the two types of light sensitive cells or proteins so as to excite and inhibit the muscle when one of the types of sensitive cells or proteins is adapted to stimulate the muscle contraction and the other is adapted to stimulate the muscle relaxation.

The muscle stimulator can be a sort of patch comprising a matrix of elements (like quantum wells, quantum dots or quantum dashes) emitting alternatively the light of the first diode λ₁ and the light of the second diode λ₂. The patch can be applied on top of the muscle to stimulate like common electro-stimulating devices. Preferably, the light-sensitive cells or proteins used are chosen so as the wavelength which can stimulate these light-sensitive cells or proteins easily penetrate in the skin and access to the muscle to stimulate without being invasive (like infrared light for example).

The present invention also relates to an artificial pacemaker for treating cardiomyopathy on a person having received an optogenetic treatment and having two types of light sensitive cells or proteins, some adapted to stimulate heart muscle contraction and others adapted to stimulate heart muscle relaxation, the artificial pacemaker comprising an apparatus as described and adapted to stimulate said two types of light sensitive cells or proteins so as to produce adequate heartbeats.

As previously described, the light-sensitive proteins used can be ChR2 proteins, which respond to blue light, and NpHR proteins, which respond to yellow light. When injected in human induced pluripotent stem cell-derived cardiomyocytes (hiPSC-CM), through optogenetic therapy, such cells become light sensitive cells.

So the association of periodic activation and inhibition of hiPSCChR2/NpHR-CM cells by the lights emitted by the apparatus can, if applied to the cells of a human heart, regulate the heart beats of someone suffering from cardiomyopathy.

In this case, the surface to treat, the heart, can be assimilated to a cavity.

The advantage of using transparent and biocompatible material as GaN in the device and apparatus is that the area, here the cavity, can be lighted and so stimulated with both sides of the semiconductor diodes.

Due to the special form of the area to treat / stimulate, the use of a vertical apparatus embodiment is preferable. However, it is also possible to use a horizontal apparatus embodiment with adjustments, like combining the lights emitted by the two diodes with a Y junction or a MultiMode Interference (MMI) coupler 230 in order to send them in the cavity with a probe 240 as shown in **Figure 12****.**

Advantageously, the artificial pacemaker further comprises a probe which collects the transmembrane voltage and sends the information to the control unit of the apparatus so that it adapts the control scheme of the diodes to equilibrate the cardiac rhythm of the patient.

The invention has many advantages. As previously written, the association of genetic introduction, interesting by its wide activation / inhibition range, and optical stimulation is very promising. It overcomes the limitation of low spatial control and homogeneity yielded by traditional electrode stimulation, which is linked in part with electrode size and spacing. Furthermore it enables a contactless stimulation that overcomes the inflammation problems due to electrode contact and can cover, with a smaller device or apparatus, an area larger than the one covered by a traditional electrode.

Although the invention has been described in the case of cardiomyopathy treatments, it can also be applied to different muscles or organs requiring an excitation and inhibition.

The description and drawings merely illustrate the principles of the invention. It will thus be appreciated that those skilled in the art will be able to devise various arrangements that, although not explicitly described or shown herein, embody the principles of the invention and are included within its spirit and scope. Furthermore, all examples recited herein are principally intended for pedagogical purposes to aid the reader in understanding the principles of the invention and the concepts contributed by the inventors to furthering the art, and are thus to be construed as being without limitation to such specifically recited examples and conditions. Moreover, all statements herein reciting principles, aspects, and embodiments of the invention, as well as specific examples thereof, are intended to encompass equivalents thereof.

### REFERENCES NUMERALS

1 Artificial Pacemaker
2 Body
3 Electrode Leads
4 Right Atrium
5 Right Ventricle
20 Blue Light
21 First hiPSC-CM colony
22 second hiPSC-CM colony
30 Yellow Light
100 Device according to a horizontal embodiment
100a First Device according to a horizontal embodiment
100b Second Device according to a horizontal embodiment
110 Device according to a vertical embodiment
110a First Device according to a vertical embodiment
110b Second Device according to a vertical embodiment
120 Substrate
125 Insulated Substrate
130 Top layer
140 Quantum well generating region / active layers
150 Insulation
160 Electrical contact
170 Insulation layer
180 Distributed Bragg Reflector
190 Power source
200 Light Emitted by the device
210a Light Emitted by the first diode at λ₁
210b Light Emitted by the second diode at λ₂
220 Passive guide
230 Y junction or MMI
240 Taper
250 Power Signal
1000 Apparatus according to a horizontal embodiment
1100 Apparatus according to a vertical embodiment
1200 Electrical assembly of an apparatus embodiment

## Claims

1. An apparatus for stimulating *in vivo* two different type of light-sensitive cells or proteins of an area comprising two devices, each devices comprising a light source, the wavelength of the light source been adapted to stimulate a type of light-sensitive cells or proteins, the first device being adapted to stimulate the first type of light-sensitive cells or proteins with a first wavelength λ₁ and the second device being adapted to stimulate the second type of light-sensitive cells or proteins with a second wavelength λ₂.

2. The apparatus according to claim 1 wherein the devices are diodes formed on the same semiconductor and electrically separated.

3. The apparatus according to claim 2 wherein the semiconductor has active layers encapsulated in a biocompatible material.

4. The apparatus according to claim 3 wherein the active layers formed a periodic structure of multi quantum wells or layers composed of quantum dots or quantum dashes generating alternatively the light of the first diode and of the second diode, the structure being adapted to optimize the homogeneity of the power density of the light emitted by the first and second diodes.

5. The apparatus according to claim 4 wherein the quantum wells or the quantum dots or quantum dashes are electrically separated.

6. The apparatus according to claim 5 wherein the semiconductor has a GaN substrate and top layer forming a p-n junction, the substrate and the top layer having opposite doping.

7. The apparatus according to claim 6 wherein the diodes are electrically polarized oppositely so that one signal is adapted to pilot them alternatively.

8. The apparatus according to claim 7 wherein the diodes generate alternatively periodical pulses.

9. The apparatus according to claim 7 or 8 further comprising a control unit adapted to modify the parameter of the signal which pilots the diodes.

10. The apparatus according to claim 9 wherein the control unit includes an input adapted to receive information coming from a probe and wherein the signal controlling the diodes is modified according to the information received.

11. The apparatus according one of the claims 2 to 10 wherein the semiconductor is insulated by an insulating layer.

12. A muscle stimulator for exciting and inhibiting a muscle having received an optogenetic treatment with two types of light sensitive cells or proteins, comprising an apparatus according to one of claims 1 to 11 adapted to stimulate the two types of light sensitive cells or proteins so as to excite and inhibit the muscle when one of the types of sensitive cells or proteins is adapted to stimulate the muscle contraction and the other is adapted to stimulate the muscle relaxation.

13. An artificial pacemaker for treating cardiomyopathy on a person having received an optogenetic treatment and having two types of light sensitive cells or proteins, comprising an apparatus according to one of claims 1 to 11 adapted to stimulate the two types of light sensitive cells or proteins so as to reproduce adapted heartbeats when one type of light sensitive cells or proteins is adapted to stimulate heart muscle contraction and the other type is adapted to stimulate heart muscle relaxation.
